# EUROPEAN PATENT APPLICATION

(11) **EP 1 944 041 A1**
(43) Date of publication of application: **16.07.2008**
(21) Application number: 06810992.5
(22) Date of filing: 26.09.2006
(51) Int. Cl.: A61K 45/00, A61K 31/05, A61K 31/565, A61K 31/566, A61K 31/573, A61K 31/58, A61P 5/30, A61P 5/36, A61P 25/00, A61P 25/02, A61P 25/04, A61P 43/00

(54) **THERAPEUTIC AGENT FOR NEUROGENIC PAIN**

(30) Priority: 06.10.2005 JP 2005294202
(71) Applicant: Japan Science and Technology Agency, Kawaguchi-shi, Saitama 332-0012 (JP); National University Corporation Tokyo Medical and Dental University, Tokyo 113-8510 (JP)
(72) Inventor: TANABE, Tsutomu, Tokyo 1510053 (JP)
(74) Representative: Helbing, Jörg
(86) International application number: PCT/JP2006/319637
(87) International publication number: WO 2007/043365

(57) **Abstract**

The present invention provides a therapeutic agent for neuropathic pain having an excellent treating effect on neuropathic pain, which is an intractable disorder. More specifically, the present invention provides a therapeutic agent for neuropathic pain and a pharmaceutical composition for treating neuropathic pain, comprising (1) a compound having an anti-progesterone activity (for example, fulvestrant (ICI182.780), fluocinolone acetonide, triamcinolone acetonide, etc.), (2) a compound having an estrogen activity (for example, 17β-estradiol), or (3) a mixture of a compound having an anti-progesterone activity and a compound having an estrogen activity (for example, 17β-estradiol and fulvestrant), as an active ingredient; a method for treating neuropathic pain using such a compound, and the like.

## Description

### TECHNICAL FIELD

The present invention relates to a therapeutic agent for neuropathic pain which has an excellent pain suppressing action against neuropathic pain, a method for treating neuropathic pain using such a therapeutic agent, and the like.

### BACKGROUND ART

Neuropathic pain is caused by, for example, injury or dysfunction in a peripheral or central nervous system, and is intractable pain for which opioid receptor agonists such as morphine are not sufficiently effective. Disorders with neuropathic pain include, for example, disorders that exhibit hyperalgesic or allodynic symptoms, such as postherpetic neuralgia, trigeminal neuralgia, diabetic neuralgia, and persistent postoperative or posttraumatic pain.

Known analgesics that have hitherto been used in conventional drug treatment include centrally acting opioid receptor agonists such as morphine and non-steroidal antiinflammatory drugs (NSAIDs) such as indomethacin. However, it is known that these analgesics generally have only a small effect on neuropathic pain, and that the effects provided by analgesics which work well for ordinary nociceptive pain (particularly, narcotic analgesics, etc.) are especially small. The inadequate analgesic effect provided by narcotic analgesics on neuropathic pain is regarded as a major characteristic of neuropathic pain. In some cases, the diagnosis of neuropathic pain is carried out using this characteristic.

Various factors are considered to be intricately involved in the onset of neuropathic pain. Conventionally known treatment methods for neuropathic pain include neurosurgical intervention such as nerve blocking and spinal epidural electrical stimulation, and lumbar intrathecal administration of drugs such as tricyclic antidepressants and baclofen. However, these treatment methods are either not sufficiently effective or have side effects. As an external preparation, capsaicin cream, which depletes the pain-producing substance P released from the nerve endings and thus alleviates pain, has been reported to be effective for postherpetic neuralgia and postmastectomy pain syndrome. However, due in part to the burning pain caused by capsaicin, the use of capsaicin cream has problems in terms of usefulness and safety. As can be seen, neuropathic pain is an intractable disorder for which an effective method of treatment has yet to be established.

ICI182.780, which is a compound having an anti-progesterone activity, is described in, for example, Japanese Patent Application (Published Japanese Translation of PCT International Publication) No. 2002-505279 as a compound used for controlling cancer (mainly, breast cancer).

It was recently reported that mifepristone (RU486: Roussel Uclaf, Paris; USP 4,386,085), which has both an anti-glucocorticoide activity and an anti-progesterone activity, exhibited an analgesic action against neuropathic pain in an experiment using neuropathic pain model mice (J. Neurosci. 24: 8595-8605 (2004); J. Neurosci. 25: 488-495 (2005)). These articles show that pain expresses in parallel to a change in the expression amount of a glucocorticoide receptor and based on this, conclude that the action of mifepristone is caused by the anti-glucocorticoide activity.

### DISCLOSURE OF THE INVENTION

As noted above, no pharmaceutical agent effective for treating neuropathic pain is known yet. Hence, such a pharmaceutical agent is desired to be developed. In light of this, it is an object of the present invention to provide a novel therapeutic agent for neuropathic pain which is highly effective against intractable pain such as neuropathic pain.

The present inventors conducted research based on their own unique ideas for achieving this object, and as a result, found that (1) a compound having an anti-progesterone activity, (2) a compound having an estrogen activity, and (3) a mixture of a compound having an anti-progesterone activity and a compound having an estrogen activity, exhibit a high analgesic effect on an intractable neuropathic pain model and thus completed the present invention.

Accordingly, the present invention provides a therapeutic agent for neuropathic pain, a pharmaceutical composition for treating neuropathic pain, a method for treating neuropathic pain and the like as follows.
(1) A therapeutic agent for neuropathic pain, comprising a compound having an anti-progesterone activity and/or an estrogen activity as an active ingredient.
(2) The therapeutic agent for neuropathic pain of (1), wherein the compound having an anti-progesterone activity is fulvestrant (ICI182.780), fluocinolone acetonide, triamcinolone acetonide, onapristone, or a pharmaceutically acceptable salt thereof.
(3) The therapeutic agent for neuropathic pain of (2), wherein the compound having an anti-progesterone activity is fulvestrant (ICI182.780) or a pharmaceutically acceptable salt thereof.
(4) The therapeutic agent for neuropathic pain of (2), wherein the compound having an anti-progesterone activity is fluocinolone acetonide or a pharmaceutically acceptable salt thereof.
(5) The therapeutic agent for neuropathic pain of (2), wherein the compound having an anti-progesterone activity is triamcinolone acetonide or a pharmaceutically acceptable salt thereof.
(6) The therapeutic agent for neuropathic pain of (1), wherein the compound having an estrogen activity is 17β-estradiol, estrone, estriol, diethylstilbestrol, or a pharmaceutically acceptable salt thereof.
(7) The therapeutic agent for neuropathic pain of (6), wherein the compound having an estrogen activity is 17β-estradiol or a pharmaceutically acceptable salt thereof.
(8) The therapeutic agent for neuropathic pain of any one of (1) through (7), wherein the neuropathic pain is at least one symptom selected from neuropathic pains in postherpetic neuralgia, trigeminal neuralgia, diabetic neuralgia, cancer pain, persistent postoperative or posttraumatic pain, hyperalgia, allodynia, postthoracotomy pain, CRPS, pain associated with multiple sclerosis, AIDS, thalamic pain, paraplegic pain caused by myelopathy, anesthesia dolorosa and phantom limb pain.
(9) A pharmaceutical composition for treating neuropathic pain, comprising a compound having an anti-progesterone activity and/or an estrogen activity and a pharmaceutically acceptable carrier.
(10) A method for treating neuropathic pain by administering an effective amount of a compound having an anti-progesterone activity and/or an estrogen activity to a mammal.
(11) Use of a compound having an anti-progesterone activity and/or an estrogen activity for producing a therapeutic agent for neuropathic pain.
(12) A therapeutic agent for neuropathic pain, comprising a combination of a compound having an anti-progesterone activity and a compound having an estrogen activity.
(13) The therapeutic agent for neuropathic pain of (12), wherein the compound having an anti-progesterone activity is fulvestrant (ICI182.780) or a pharmaceutically acceptable salt thereof, and the compound having an estrogen activity is 17β-estradiol, estrone, estriol, diethylstilbestrol, or a pharmaceutically acceptable salt thereof.
(14) The therapeutic agent for neuropathic pain of (13), wherein the compound having an anti-progesterone activity is fulvestrant (ICI182.780) or a pharmaceutically acceptable salt thereof, and the compound having an estrogen activity is 17β-estradiol or a pharmaceutically acceptable salt thereof.

A therapeutic agent for neuropathic pain according to the present invention is effective for the treatment of neuropathic pain which exhibits symptoms such as postherpetic neuralgia, trigeminal neuralgia, diabetic neuralgia, cancer pain, persistent postoperative or posttraumatic pain, hyperalgia, allodynia and the like.

### BRIEF DESCRIPTION OF THE DRAWINGS

FIG. 1 shows experimental results in Example 1, illustrating a change in the pain threshold against mechanical stimulation exhibited by pain-hypersensitive rats to which ICI182.780 was intraperitoneally administered.
FIG. 2 shows experimental results in Example 2, illustrating a change in the pain threshold against mechanical stimulation exhibited by pain-hypersensitive rats to which 17β-estradiol was intraperitoneally administered.
FIG. 3 shows experimental results in Example 3, illustrating a change in the pain threshold against mechanical stimulation exhibited by pain-hypersensitive rats to which a mixture of an equal amount of 17β-estradiol and ICI182.780 was intraperitoneally administered.
FIG. 4 shows experimental results in Example 4, illustrating a change in the pain threshold against thermal stimulation exhibited by pain-hypersensitive rats to which a mixture of an equal amount of 17β-estradiol and ICI182.780 was intraperitoneally administered.
FIG. 5 shows experimental results in Example 5, illustrating a change in the pain threshold against mechanical stimulation exhibited by pain-hypersensitive rats to which fluocinolone acetonide was intraperitoneally administered.
FIG. 6 shows experimental results in Example 6, illustrating a change in the pain threshold against thermal stimulation exhibited by pain-hypersensitive rats to which fluocinolone acetonide was intraperitoneally administered.
FIG. 7 shows experimental results in Example 7, illustrating a change in the pain threshold against mechanical stimulation exhibited by pain-hypersensitive rats to which triamcinolone acetonide was intraperitoneally administered.

### BEST MODE FOR CARRYING OUT THE INVENTION

Hereinafter, the present invention will be described in more detail.

The present invention provides a therapeutic agent for neuropathic pain, comprising a compound having an anti-progesterone activity and/or an estrogen activity as an active ingredient; a pharmaceutical composition for treating neuropathic pain, comprising a compound having an anti-progesterone activity and/or an estrogen activity and a pharmaceutically acceptable carrier; a method for treating neuropathic pain using a compound having an anti-progesterone activity and/or an estrogen activity; a therapeutic agent for neuropathic pain, comprising a mixture of a compound having an anti-progesterone activity and a compound having an estrogen activity as an active ingredient; and the like.

Various types of compounds having an anti-progesterone activity and compounds having an estrogen activity are known, but no report in the past has discussed an analgesic effect of such a compound or a combination of such compounds in a neuropathic pain model. The present inventor found for the first time in history that, surprisingly, such a compound has an effect of treating neuropathic pain independently and furthermore that a mixture of a compound having an anti-progesterone activity and a compound having an estrogen activity has a stronger treating effect than such a compound used independently.

As used herein, the term "compound having an anti-progesterone activity" refers to a substance having an action of inhibiting or suppressing a progesterone activity. The term "progesterone activity" refers to an activity that, for example, stimulates a lipoprotein lipase activity to promote accumulation of lipid, or exhibits a calmative or hypnotic action in the central nervous system. It can be confirmed that an agent has an anti-progesterone activity by known methods, for example, a method described in CANCER RESEARCH, Vol. 59, 372-376, 1999. Estrogen is used in a mixed oral contraceptive or for post-menopausal hormone replacement therapy. The term "estrogen activity" refers to an activity that, for example, causes many of the changes explaining female secondary sex characteristics, decreases the number and activity of osteoclast, increases the amount of high density lipoprotein (HDL) or causes low density lipoprotein (LDL) and lipoprotein a (LPa), or an activity that carries out transcriptional control by increasing or decreasing the synthesis of mRNA from a target gene. It can be confirmed that an agent has an estrogen activity by known methods, for example, a method described in Mol. Cell Biol., Vol.. 25, 5417-5428, 2005.

As used herein, the term "treatment" generally means improving the symptoms of humans and mammals other than humans. The term "improvement" refers to, for example, alleviating, or preventing exacerbation of, the severity of a disorder as compared with the case where a therapeutic agent of the present invention is not administered. This term also has the meaning of prophylaxis. The term "pharmaceutical composition" refers to a composition containing an active ingredient useful for the present invention (ICI182.780, 17β-estradiol, fluocinolone acetonide, triamcinolone acetonide, etc.) and an additive such as a carrier or the like used in preparing a pharmaceutical agent.

Examples of the compound having an anti-progesterone activity used in the present invention include fulvestrant (ICI182.780), fluocinolone acetonide, triamcinolone acetonide, onapristone, pharmaceutically acceptable salts thereof, and the like.

Preferable examples of the compound having an anti-progesterone activity used in the present invention include fulvestrant (ICI182.780), pharmaceutically acceptable salts thereof, and the like. Fulvestrant (ICI182.780) has a compound name of 7α-17β-[9-[(4,4,5,5,5-pentafluoropentyl)sulphinyl]nonyl]estra-1,3,5(10)-trien-3,17-diol, and has an anti-estrogen activity as well as an anti-progesterone activity (see CANCER RESEARCH, Vol. 59, 372-376, 1999; Japanese Patent Application (Published Japanese Translation of PCT International Publication) No. 2002-505279; Japanese Patent Application (Published Japanese Translation of PCT International Publication) No. 10-511378; etc.). Fulvestrant (ICI182.780) was approved as a therapeutic agent for hormone receptor-positive recurrent breast cancer (trade name: Faslodex; AstraZeneca Pharmaceuticals LP) in April 2002 by FDA.

Examples of the compound exhibiting an estrogen activity used in the present invention include 17β-estradiol, estrone, estriol, diethylstilbestrol, and the like.

The above-listed compounds are all known. For example, fulvestrant (ICI182.780) and fluocinolone acetonide are respectively registered as CAS Nos. 129453-61-8 and 67-73-2. These compounds are described in The Merck Index, 13th Edition (2001), Wako Pure Chemical Industries, Ltd. Comprehensive Catalog 2004, SIGMA Comprehensive Catalog (2004-2005), The Pharmacological Basis of Therapeutics 10th Edition (McGraw Hill), and the like. For example, 17β-estradiol, triamcinolone acetonide and fluocinolone acetonide are respectively described on pages 3738, 9671 and 734-735 of The Merck Index regarding, for example, the compound names, chemical structures, and physicochemical properties thereof, and main documents relevant thereto. Fulvestrant (ICI182.780) is described in the Japanese language version of The Pharmacological Basis of Therapeutics mentioned above (Hirokawa Shoten, 2003), pages 2059-2060 regarding, for example, the compound name, chemical structure, and functional mechanisms thereof.

As used herein, the expression "comprising a compound having an anti-progesterone activity and/or an estrogen activity" is used to encompass all the uses of any compound having an anti-progesterone activity and/or an estrogen activity in any pharmaceutically acceptable form (for example, in salt, ester, amide, hydrate or solvate state, racemic mixture, optically pure form, prodrug, or the like thereof).

Accordingly, the compound used as an active ingredient in the present invention may be a free compound or a pharmaceutically acceptable salt. Such a "salt" encompasses acid salts and basic salts. Examples of the acid salts include hydrochlorides, hydrobromides, hydroiodides, nitrates, sulfates, bisulfates, phosphates, acid phosphates, acetates, lactates, citrates, acid citrates, tartrates, bitartrates, succinates, maleates, fumarates, gluconates, saccharates, benzoates, methanesulfonates, ethanesulfonates, benzenesulfonates, p-toluenesulfonates, 1,1'-methylene-bis-(2-hydroxy-3-naphthoic acid) salt, and the like. Examples of the basic salts include alkaline metal salts such as sodium salts, potassium salts, and the like; alkaline earth metal salts such as calcium salts, magnesium salts and the like; water-soluble amine addition salts such as ammonium salts, N-methylglucaminates and the like; lower alkanol ammonium salts; and salts derived from the other pharmaceutically acceptable organic amine bases.

A therapeutic agent and a composition for neuropathic pain according to the present invention are effective for treating neuropathic pain. Examples of the neuropathic pain include neuropathic pains in postherpetic neuralgia, trigeminal neuralgia, diabetic neuralgia, cancer pain, persistent postoperative or posttraumatic pain, hyperalgia, allodynia, postthoracotomy pain, CRPS, pain associated with multiple sclerosis, AIDS, thalamic pain, paraplegic pain caused by myelopathy, anesthesia dolorosa, phantom limb pain, and the like. A therapeutic agent for neuropathic pain according to the present invention is especially effective for treating hyperalgia and allodynia.

A therapeutic agent for neuropathic pain according to the present invention may be administered orally or parenterally with no specific limitation on the manner of administration. Active ingredients of the therapeutic agent for neuropathic pain according to the present invention may be provided independently or in a combination, or provided as being contained in a formulation together with a pharmaceutically acceptable carrier or an additive for formulation. In the latter case, the active ingredient of the present invention may be contained in, for example, 0.1 to 99.9% by weight with respect to the entirety of the formulation.

Examples of the pharmaceutically acceptable carrier or additive usable in the present invention include excipient, disintegrator, disintegration aid, binder, lubricant, coating agent, colorant, diluent, dissolving agent, dissolution aid, tonicity agent, pH modifier, stabilizer, and the like.

Examples of the form of formulation suitable to oral administration include powder, tablet, capsule, fine granule, granule, liquid, syrup, and the like. For oral administration, any of various excipients such as microcrystalline cellulose, sodium citrate, calcium carbonate, dipotassium phosphate, glycine or the like may be used together with any of various disintegrators such as starch (preferably corn, potato or tapioca starch), alginic acid, a certain type of silicate double salt or the like; and a granule-forming binder such as polyvinylpyrrolidone, sucrose, gelatin, gum arabic or the like. A lubricant such as magnesium stearate, sodium lauryl sulfate, talc or the like is often very effective for tablet formation. A gelatin capsule filled with the same type of solid composition may be used. Substances preferably usable in connection with this include lactose as well as high-molecular-weight polyethyleneglycol. For preparing an aqueous suspension and/or elixir for oral administration, the active ingredient may be used together with any of various types of sweeteners, flavorings, coloring agents or dyes, optionally an emulsifier and/or a suspending agent, as well as a diluent such as water, ethanol, propyleneglycerol, glycerol, or the like or a combination thereof.

Examples of the form of formulation suitable to parenteral administration include injection, suppository, and the like. For parenteral administration, the active ingredient of the present invention may be dissolved in either sesame oil or peanut oil, or in an aqueous solution of propyleneglycol. When necessary, the aqueous solution is appropriately buffered (preferably to pH8 or higher) to first isotonize the liquid diluent. Such an aqueous solution is suitable for intravenous injection, and an oleaginous solution is suitable for intraarticular injection, intramuscular injection and subcutaneous injection. All of these solutions can easily be produced under aseptic conditions by a standard pharmaceutical technology well known to those skilled in the art. In addition, the active ingredient of the present invention may be topically administered to skin or the like. In such a case, it is desirable to topically administer the active ingredient in the form of cream, jelly, paste or ointment in accordance with the standard pharmaceutical practice.

A therapeutic agent for neurophathic pain according to the present invention may be administered in an appropriate dose, with no specific limitation, which is selected in accordance with various conditions including the type of pain, age or symptom of the patient, administration route, purpose of treatment, and presence or absence of another medication used together with the agent. A daily dose of the therapeutic agent for neuropathic pain according to the present invention is, for example, about 500 to about 25,000 mg, and preferably 900 to 9000 mg, for an adult (e.g., body weight: 60 kg). When administered as an injection, a daily dose is about 100 to about 5,000 mg, and preferably 180 to 1,800 mg, for an adult (e.g., body weight: 60 kg). Such a daily dose may be divided into 2 to 4 separate doses.

### EXAMPLES

Hereinafter, the present invention will be described more specifically by way of examples, which are not intended to limit the scope of the present invention.

### (Experimental materials and general experimental method)

### (1) Model animals

As experimental animals, pain hypersensitivity model rats obtained by conducting complete ligation on L5/L6 spinal nerves of 6-week-old male rats were used.

### (2) Grouping

Mechanical stimulation tests were carried out using Dynamic Plantar Aesthesiometer (37400, Ugo Basile), and thermal stimulation tests were carried out using a plantar thermal stimulation tester (Plantar Test 7370, Ugo Basile). The pain threshold of the feet of each model animal was measured, and the animals were divided into groups using Preclinical Package Version 5.0 (SAS Institute Japan), such that the pain thresholds are uniform among the groups as measured prior to administration on each day of the experiment. In mechanical stimulation, model animals exhibiting a foot pain threshold of 8.0 g or more were excluded from the tests. In thermal stimulation, model animals exhibiting a foot pain threshold of 10 seconds or more were excluded from the tests.

### (3) Preparation of test substance

The test substance was prepared as follows. The base material was ground with an agate mortar and a pestle, and 0.5 w/v% carboxymethylcellulose sodium (CMC-Na) as a medium was gradually added thereto to form a uniform suspension. The concentration of the liquid to be administered was adjusted with a graduated cylinder or a volumetric flask, only before use.

### (4) Method of administration

The purpose of administering the test substance is to ascertain the direct effect on spinal cord. Since the test substance had been confirmed to pass through the brain barrier, a simple intraperitoneal administration was used. The test substance in a volume of 5 ml/kg was intraperitoneally administered using a syringe barrel and a needle.

### Example 1

### (ICI128.780, mechanical stimulation method)

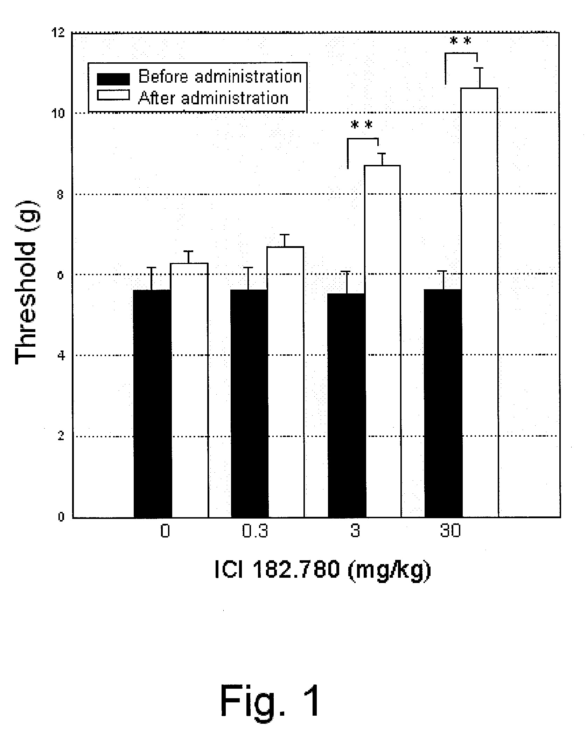

Groups each including five pain hypersensitivity model male rats (355.8 to 457.9 g) were used. Before administration of ICI182.780, and at 30 minutes, 60 minutes and 90 minutes after administration of ICI182.780, the pain threshold of the left plantar of each rat was measured using a stimulating apparatus which had been set such that the maximum pressure would be 15.0 g and the maximum pressure would be reached in 20 seconds. The results are shown in FIG. 1. In FIG. 1, "**" indicates that there is a significant difference at P < 0.01 based on the Dunnett's multiple comparison test, and "*" indicates that there is a significant difference at P < 0.05 based on the Dunnett's multiple comparison test (this is also applied to the following).

As shown in FIG. 1, the control group to which a 0.5 w/v% aqueous solution of carboxymethylcellulose sodium (CMC-Na) was administered exhibited a maximum pain threshold of 6.3 g after the administration, whereas the group to which ICI182.780 was administered exhibited the following results: (a) after the administration of 0.3 mg/kg, the maximum pain threshold was 6.7 g; (b) after the administration of 3 mg/kg, the maximum pain threshold was 8.7 g; and (c) after the administration of 30 mg/kg, the maximum pain threshold was 10.6 g. As understood from these results, the administration ofICI182.780 significantly increased the pain threshold. This confirmed the analgesic effect of ICI182.780 on neuropathic pain.

### Example 2

### (17β-estradiol, mechanical stimulation method)

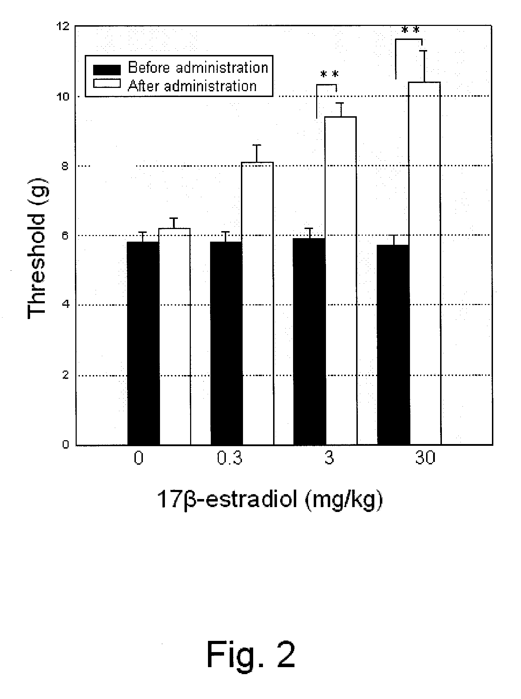

Groups each including five pain hypersensitivity model male rats (290.3 to 354.8 g) were used. Before administration of 17β-estradiol, and at 30 minutes, 60 minutes and 90 minutes after administration of 17β-estradiol, the pain threshold of the left plantar of each rat was measured using a stimulating apparatus which had been set such that the maximum pressure would be 15.0 g and the maximum pressure would be reached in 20 seconds. The results are shown in FIG. 2.

As shown in FIG. 2, the control group to which a 0.5 w/v% aqueous solution of carboxymethylcellulose sodium (CMC-Na) was administered exhibited a maximum pain threshold of 6.2 g after the administration, whereas the group to which 17β-estradiol was administered exhibited the following results: (a) after the administration of 0.3 mg/kg, the maximum pain threshold was 8.1 g; (b) after the administration of 3 mg/kg, the maximum pain threshold was 9.4 g; and (c) after the administration of 30 mg/kg, the maximum pain threshold was 10.4 g. As understood from these results, the administration of 17β-estradiol significantly increased the pain threshold. This confirmed the analgesic effect of 17β-estradiol on neuropathic pain.

### Example 3

### (17β-estradiol + ICI128.780, mechanical stimulation method)

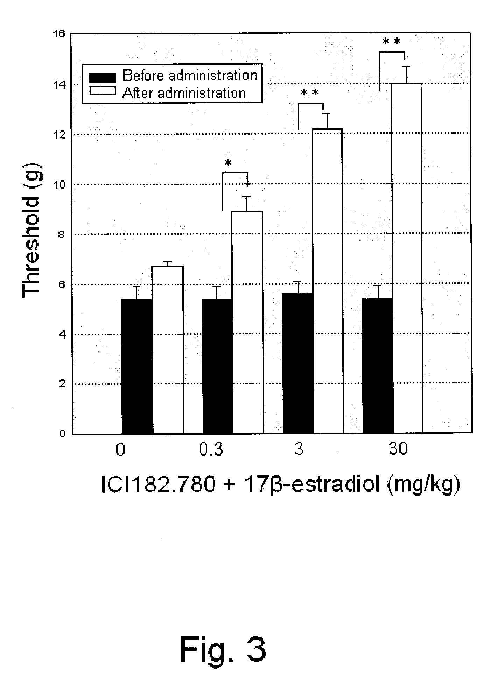

Groups each including five pain hypersensitivity model male rats (294.8 to 415.4 g) were used. Before administration of 17β-estradiol + ICI128.780, and at 30 minutes, 60 minutes and 90 minutes after administration of 17β-estradiol + ICI182.780, the pain threshold of the left plantar of each rat was measured using a stimulating apparatus which had been set such that the maximum pressure would be 15.0 g and the maximum pressure would be reached in 20 seconds. The results are shown in FIG. 3.

As shown in FIG. 3, the control group to which a 0.5 w/v% aqueous solution of carboxymethylcellulose sodium (CMC-Na) was administered exhibited a maximum pain threshold of 6.7 g after the administration, whereas the group to which 17β-estradiol + ICI128.780 was administered exhibited the following results: (a) after the administration of 0.3 mg/kg, the maximum pain threshold was 8.9 g; (b) after the administration of 3 mg/kg, the maximum pain threshold was 12.2 g; and (c) after the administration of 30 mg/kg, the maximum pain threshold was 14.0 g. As understood from these results, the administration of 17β-estradiol + ICI128.780 significantly increased the pain threshold. This confirmed the analgesic effect of 17β-estradiol + ICI182.780 on neuropathic pain. In the above-described pain hypersensitivity models, the pain threshold was lowered conspicuously due to allodynia, by which tactile stimulus which is not usually felt as pain is felt as pain. Then, the administration of 17β-estradiol + ICI128.780 increased the pain threshold in a dose-dependent manner when intraperitoneally administered and was confirmed to improve the symptom of pain hypersensitivity.

### Example 4

### (17β-estradiol + ICI128.780, thermal stimulation method)

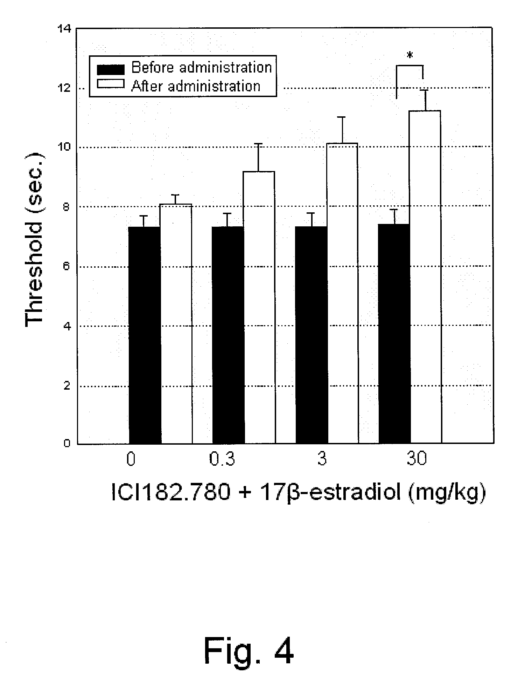

Groups each including five pain hypersensitivity model male rats (282.0 to 452.4 g) were used. Before administration of 17β-estradiol + ICI128.780, and at 30 minutes, 60 minutes and 90 minutes after administration of 17β-estradiol + ICI182.780, the pain threshold of the left plantar of each rat was measured using a plantar thermal stimulating apparatus set to a thermal stimulation intensity of 45. The results are shown in FIG. 4.

As shown in FIG. 4, the control group to which a 0.5 w/v% aqueous solution of carboxymethylcellulose sodium (CMC-Na) was administered exhibited a maximum pain threshold of 8.1 seconds after the administration, whereas the group to which 17β-estradiol + ICI128.780 was administered exhibited the following results: (a) after the administration of 0.3 mg/kg, the maximum pain threshold was 9.2 seconds; (b) after the administration of 3 mg/kg, the maximum pain threshold was 10.1 seconds; and (c) after the administration of 30 mg/kg, the maximum pain threshold was 11.2 seconds. As understood from these results, the administration of 17β-estradiol + ICI128.780 significantly increased the pain threshold. This confirmed the analgesic effect of 17β-estradiol + ICI182.780 on neuropathic pain.

### Example 5

### (Fluocinolone acetonide, mechanical stimulation method)

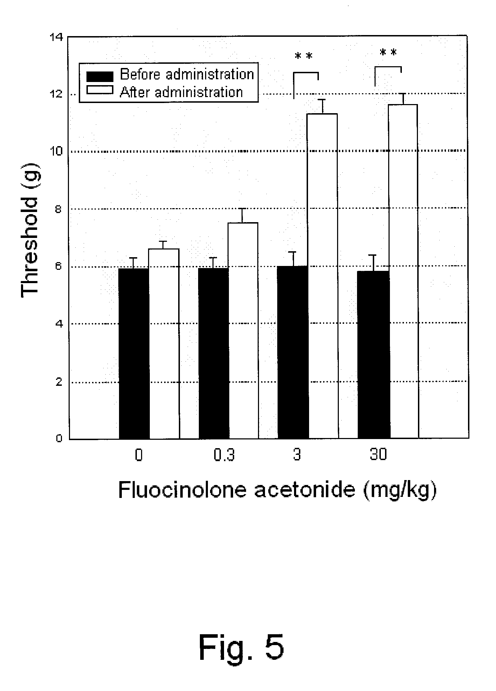

Groups each including five pain hypersensitivity model male rats (333.7 to 408.3 g) were used. Before administration of fluocinolone acetonide, and at 30 minutes, 60 minutes and 90 minutes after administration of fluocinolone acetonide, the pain threshold of the left plantar of each rat was measured using a stimulating apparatus which had been set such that the maximum pressure would be 15.0 g and the maximum pressure would be reached in 20 seconds. The results are shown in FIG. 5.

As shown in FIG. 5, the control group to which a 0.5 w/v% aqueous solution of carboxymethylcellulose sodium (CMC-Na) was administered exhibited a maximum pain threshold of 6.6 g after the administration, whereas the group to which fluocinolone acetonide was administered exhibited the following results: (a) after the administration of 0.3 mg/kg, the maximum pain threshold was 7.5 g; (b) after the administration of 3 mg/kg, the maximum pain threshold was 11.3 g; and (c) after the administration of 10 mg/kg, the maximum pain threshold was 11.6 g. As understood from these results, the administration of fluocinolone acetonide significantly increased the pain threshold. This confirmed the analgesic effect of fluocinolone acetonide on neuropathic pain. In the above-described pain hypersensitivity models, the pain threshold was lowered conspicuously due to allodynia, by which tactile stimulus which is not usually felt as pain is felt as pain. Then, the administration of fluocinolone acetonide increased the pain threshold in a dose-dependent manner when intraperitoneally administered and was confirmed to improve the symptom of pain hypersensitivity.

### Example 6

### (Fluocinolone acetonide, thermal stimulation method)

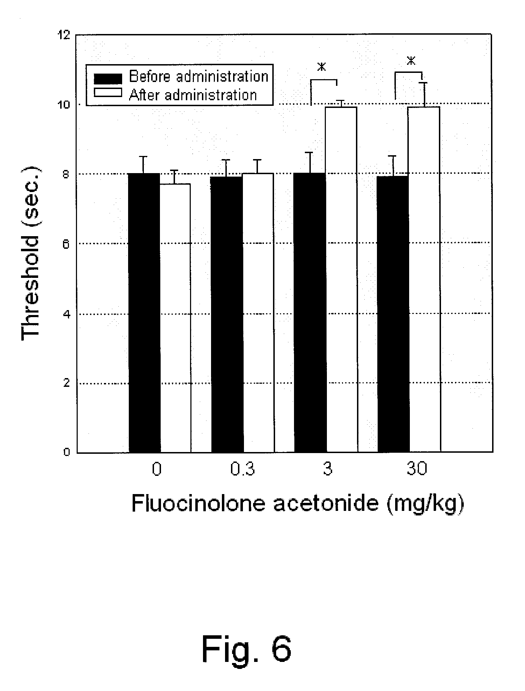

Groups each including five pain hypersensitivity model male rats (320.0 to 449.6 g) were used. Before administration of fluocinolone acetonide, and at 30 minutes, 60 minutes and 90 minutes after administration of fluocinolone acetonide, the pain threshold of the left plantar of each rat was measured using a plantar thermal stimulating apparatus set to a thermal stimulation intensity of 35. The results are shown in FIG. 6.

As shown in FIG. 6, the control group to which a 0.5 w/v% aqueous solution of carboxymethylcellulose sodium (CMC-Na) was administered exhibited a maximum pain threshold of 7.7 seconds after the administration, whereas the group to which fluocinolone acetonide was administered exhibited the following results: (a) after the administration of 0.3 mg/kg, the maximum pain threshold was 8.0 seconds; (b) after the administration of 3 mg/kg, the maximum pain threshold was 9.9 seconds; and (c) after the administration of 10 mg/kg, the maximum pain threshold was 9.9 seconds. As understood from these results, the administration of fluocinolone acetonide significantly increased the pain threshold. This confirmed the analgesic effect of fluocinolone acetonide on neuropathic pain.

### Example 7

### (Triamcinolone acetonide, mechanical stimulation method)

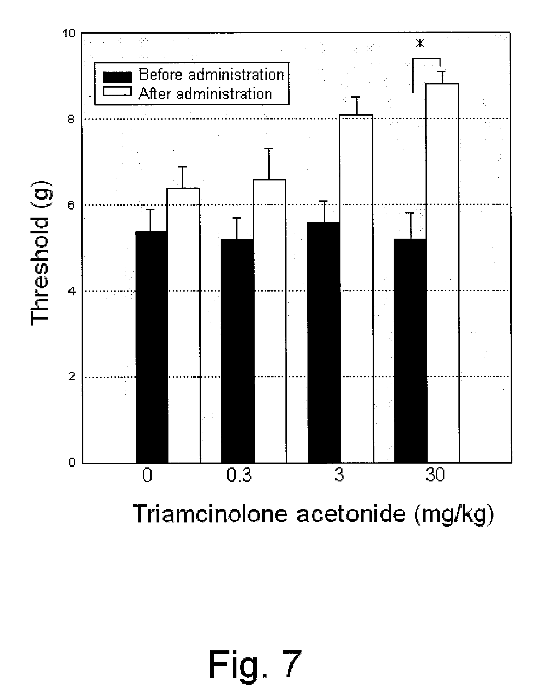

Groups each including five pain hypersensitivity model male rats (261.4 to 320.9 g) were used. Before administration of triamcinolone acetonide, and at 30 minutes, 60 minutes and 90 minutes after administration of triamcinolone acetonide, the pain threshold of the left plantar of each rat was measured using a stimulating apparatus which had been set such that the maximum pressure would be 15.0 g and the maximum pressure would be reached in 20 seconds. The results are shown in FIG. 7.

As shown in FIG. 7, the control group to which a 0.5 w/v% aqueous solution of carboxymethylcellulose sodium (CMC-Na) was administered exhibited a maximum pain threshold of 6.4 g after the administration, whereas the group to which triamcinolone acetonide was administered exhibited the following results: (a) after the administration of 0.3 mg/kg, the maximum pain threshold was 6.6 g; (b) after the administration of 3 mg/kg, the maximum pain threshold was 8.1 g; and (c) after the administration of 30 mg/kg, the maximum pain threshold was 8.8 g. As understood from these results, the administration of triamcinolone acetonide significantly increased the pain threshold. This confirmed the analgesic effect of triamcinolone acetonide on neuropathic pain. In the above-described pain hypersensitivity models, the pain threshold was lowered conspicuously due to allodynia, by which tactile stimulus which is not usually felt as pain is felt as pain. Then, triamcinolone acetonide increased the pain threshold in a dose-dependent manner when intraperitoneally administered and was confirmed to improve the symptom of pain hypersensitivity.

### Discussion

The above-described examples revealed that a therapeutic agent for neuropathic pain comprising (1) a compound having an anti-progesterone activity, (2) a compound having an estrogen activity or (3) a mixture of a compound having an anti-progesterone activity and a compound having an estrogen activity, as an active ingredient is effective for treating neuropathic pain.

### INDUSTRIAL APPLICABILITY

As described above, a therapeutic agent for neuropathic pain comprising (1) a compound having an anti-progesterone activity, (2) a compound having an estrogen activity or (3) a mixture of a compound having an anti-progesterone activity and a compound having an estrogen activity, as an active ingredient has an action of improving symptoms of neuropathic pain caused by various reasons and therefore can be effectively used for treating neuropathic pain.

## Claims

1. A therapeutic agent for neuropathic pain, comprising a compound having an anti-progesterone activity and/or an estrogen activity as an active ingredient.

2. The therapeutic agent for neuropathic pain of claim 1, wherein the compound having an anti-progesterone activity is fulvestrant (ICI182.780), fluocinolone acetonide, triamcinolone acetonide, onapristone, or a pharmaceutically acceptable salt thereof.

3. The therapeutic agent for neuropathic pain of claim 2, wherein the compound having an anti-progesterone activity is fulvestrant (ICI182.780) or a pharmaceutically acceptable salt thereof.

4. The therapeutic agent for neuropathic pain of claim 2, wherein the compound having an anti-progesterone activity is fluocinolone acetonide or a pharmaceutically acceptable salt thereof.

5. The therapeutic agent for neuropathic pain of claim 2, wherein the compound having an anti-progesterone activity is triamcinolone acetonide or a pharmaceutically acceptable salt thereof.

6. The therapeutic agent for neuropathic pain of claim 1, wherein the compound having an estrogen activity is 17β-estradiol, estrone, estriol, diethylstilbestrol, or a pharmaceutically acceptable salt thereof.

7. The therapeutic agent for neuropathic pain of claim 6, wherein the compound having an estrogen activity is 17β-estradiol or a pharmaceutically acceptable salt thereof.

8. The therapeutic agent for neuropathic pain of any one of claims 1 through 7, wherein the neuropathic pain is at least one symptom selected from neuropathic pains in postherpetic neuralgia, trigeminal neuralgia, diabetic neuralgia, cancer pain, persistent postoperative or posttraumatic pain, hyperalgia, allodynia, postthoracotomy pain, CRPS, pain associated with multiple sclerosis, AIDS, thalamic pain, paraplegic pain caused by myelopathy, anesthesia dolorosa and phantom limb pain.

9. A pharmaceutical composition for treating neuropathic pain, comprising a compound having an anti-progesterone activity and/or an estrogen activity and a pharmaceutically acceptable carrier.

10. A method for treating neuropathic pain by administering an effective amount of a compound having an anti-progesterone activity and/or an estrogen activity to a mammal.

11. Use of a compound having an anti-progesterone activity and/or an estrogen activity for producing a therapeutic agent for neuropathic pain.

12. A therapeutic agent for neuropathic pain, comprising a combination of a compound having an anti-progesterone activity and a compound having an estrogen activity.

13. The therapeutic agent for neuropathic pain of claim 12, wherein the compound having an anti-progesterone activity is fulvestrant (ICI182.780) or a pharmaceutically acceptable salt thereof, and the compound having an estrogen activity is 17β-estradiol, estrone, estriol, diethylstilbestrol, or a pharmaceutically acceptable salt thereof.

14. The therapeutic agent for neuropathic pain of claim 13, wherein the compound having an anti-progesterone activity is fulvestrant (ICI182.780) or a pharmaceutically acceptable salt thereof, and the compound having an estrogen activity is 17β-estradiol or a pharmaceutically acceptable salt thereof.
